# EUROPEAN PATENT SPECIFICATION

(11) **EP 2 090 318 B1**
(45) Date of publication and mention of the grant of the patent: **08.08.2012**
(21) Application number: 07822990.3
(22) Date of filing: 17.10.2007
(51) Int. Cl.: A61K 39/04, A61P 37/04

(54) **PROPHYLACTIC TUBERCULOSIS VACCINE**
PROPHYLAKTISCHER TUBERKULOSEIMPFSTOFF
VACCIN PROPHYLACTIQUE CONTRE LA TUBERCULOSE

(30) Priority: 30.10.2006 ES 200602754
(43) Date of publication of application: 19.08.2009
(73) Proprietor: Archivel Farma, SL, 08916 BADALONA (ES)
(72) Inventor: CARDONA IGLESIAS, Pere Joan, 08916 Badalona (Barcelona) (ES); AMAT RIERA, Isabel, 08916 Badalona (Barcelona) (ES)
(74) Representative: HOFFMANN EITLE
(86) International application number: PCT/ES2007/000583
(87) International publication number: WO 2008/053055

(56) References cited:
- EP-A- 1 098 199
- EP-A- 1 690 549
- WO-A1-03/063897
- WO-A2-00/21983
- ES-A1- 2 231 037
- US-A1- 2002 127 700
- US-A1- 2002 176 867
- AGGER E M ET AL: "Specific acquired resistance in mice immunized with killed mycobacteria." SCANDINAVIAN JOURNAL OF IMMUNOLOGY NOV 2002, vol. 56, no. 5, November 2002 (2002-11), pages 443-447, XP002560801 ISSN: 0300-9475
- CHATURVEDI V ET AL: "Protective antigens of Mycobacterium habana are distributed between peripheral and integral compartments of plasma membrane: a study in experimental tuberculosis of mouse" VACCINE, BUTTERWORTH SCIENTIFIC. GUILDFORD, GB, vol. 17, no. 22, 16 July 1999 (1999-07-16), pages 2882-2887, XP004171721 ISSN: 0264-410X
- DATABASE WPI Week 200102, Derwent Publications Ltd., London, GB; AN 2001-014156, XP003022729 & RU 2 153 354 C1 (IMMUNOL. INST. RES. CENTRE ET AL.) 27 July 2000
- SINHA S. ET AL.: 'Proteome Analysis of the Plasma Membrane of Mycobacterium tuberculosis' COMPARATIVE AND FUNCTIONAL GENOMICS vol. 3, 2002, pages 470 - 483, XP002998683
- GREGORIADIS G ET AL: J CONTROLLED RELEASE, vol. 41, 1996, pages 49-56,
- Sigma-Aldrich: "Product Information Dimethyldioctadecylammonium bromide", , Retrieved from the Internet: URL:http://www.sigmaaldrich.com/etc/medial ib/docs/Sigma/Product_Information_Sheet/d2 779pis.Par.0001.File.tmp/d2779pis.pdf [retrieved on 2010-08-02]
- COMAS IÑAKI ET AL: "Human T cell epitopes of Mycobacterium tuberculosis are evolutionarily hyperconserved.", NATURE GENETICS JUN 2010 LNKD- PUBMED:20495566, vol. 42, no. 6, June 2010 (2010-06), pages 498-503, ISSN: 1546-1718
- ANDERSEN P: "Effective vaccination of mice against Mycobacterium tuberculosis infection with a soluble mixture of secreted mycobacterial proteins.", INFECTION AND IMMUNITY JUN 1994 LNKD- PUBMED:7910595, vol. 62, no. 6, June 1994 (1994-06), pages 2536-2544, ISSN: 0019-9567

## Description

### Field of the Art

The present invention relates to the use of an immunotherapeutic agent based on cell wall fragments of a virulent strain of *Mycobacterium tuberculosis*-complex for the preparation of a drug for the prophylactic treatment of tuberculosis.

### Prior State of the Art

Tuberculosis is a chronic infectious disease caused by the *Mycobacterium tuberculosis-*complex (MTB-C) bacilli, which currently include the species *M. tuberculosis, M. bovis, M. microti* and *M. africanum.*

According to the World Health Organization, 8,000,000 new cases of people manifesting the disease are recorded worldwide every year and about 3,000,000 people die. It is considered that there are more than 2,000,000,000 infected people worldwide and that 90-100 million more new infections are generated each year.

The current vaccine which is used in the preventive treatment against tuberculosis is based on bacteria of the strain called BCG (Bacillus Calmette-Guerin), an attenuated variant of *M. bovis*.

Various vaccines against tuberculosis based on cell wall fragments of virulent or avirulent strains of *Mycobacterium* are described in the state of the art. It is also described that the adjuvant used in the composition of the vaccine greatly influences the effectiveness thereof.

E. Ribi et al., Nature 1963, 198, pages 1214 to 1215, describe the immunization assays performed with a composition comprising cell wall fragments of the avirulent BCG strain and mineral oil. Said fragments are obtained by homogenisation of a culture of the mentioned strain in mineral oil. The composition is more effective than the conventional vaccine (BCG). Nevertheless, it is described in the same article that the cell wall fragments do not induce any immunological response when they are obtained by homogenisation in water and in the absence of the mineral oil.

D.P. Pal et al., Indian J. Med. Res. 1977, 65, pages 340 to 345, describe a vaccine prepared with cell wall fragments of the virulent H₃₇Rv strain and mineral oil. In this case the cell wall fragments are obtained by means of homogenisation of the dead cells in aqueous phase, and the mineral oil is subsequently added to the composition. It is also described that the cell wall fragments homogenised in aqueous phase are not immunogenic and that the presence of mineral oil is necessary for the vaccine to be effective.

G.K. Khuller et al., Folia Microbiol., 1992, 37, pages 407 to 412, describe the protective efficacy of different fractions of the cell wall of the avirulent H₃₇Ra strain of *M. tuberculosis* formulated with Freund's incomplete adjuvant, which also includes mineral oil.

E.M. Agger et al., Scand. J. Immunol., 2002, 56, pages 443 to 447, describe vaccines comprising cell wall fragments of the virulent H₃₇Rv strain, which are effective when they include the cationic surfactant dimethyldioctadecylammonium bromide as an adjuvant. It is also described that the assays conducted with homogenised *M. tuberculosis* bacilli which do not contain the mentioned adjuvant do not generate levels of resistance against tuberculosis in the murine model.

I.M. Orme Vaccine, 2006, 24, pages 2 to 19, which is a recent review article of new vaccines against tuberculosis, describes that the conventional BCG vaccine is essentially ineffective in protecting adult people against tuberculosis. It is indicated in the same article that several candidates for different types of vaccines (subunit vaccines with proteins, vaccines with DNA, vaccines combined with virus, vaccines with recombinant strains) are being assayed and that new developments are expected.

It is therefore necessary to have a prophylactic vaccine to prevent infections caused by *M. tuberculosis* that is more effective than the current vaccine based on the attenuated BCG strain.

### Object of the Invention

The object of the present invention is the use of an immunotherapeutic agent consisting essentially of cell wall fragments of a virulent strain of MTB-C in the form of liposomes for the preparation of a drug for the prophylactic treatment of infections caused by *M. tuberculosis.*

### Detailed Description of the Invention

Patent application ES2231037-A1 discloses a method for the preparation of an immunotherapeutic agent comprising cell wall fragments of a virulent strain of *Mycobacterium tuberculosis*-complex (MTB-C). It also discloses compositions containing it and the therapeutic application thereof for the combined treatment of tuberculosis in association with other drugs.

The authors of the present invention have discovered the use of said immunotherapeutic agent for the preparation of a drug for the prophylactic treatment of tuberculosis.

The object of the present invention therefore is the use of an immunotherapeutic agent consisting essentially of cell wall fragments of a virulent strain of *Mycobacterium tuberculosis-*complex (MTB-C) for the preparation of a drug for the prophylactic treatment of tuberculosis, whereinsaid agent is obtainable by a method comprising the following steps:
- cultivate the virulent MTB-C strain over a period equal to or greater than three weeks and, subsequently,
- homogenate the cell culture in the presence of a nonionic surfactant,
- separating the non-fragmented cells and the solubilized components by means of centrifugation,
- subjecting the fraction of cell wall fragments to chemical or physical treatment to inactivate the eventual virulent strain cells that it eventually contains, and
- drying the obtained immunotherapeutic agent by means of lyophilisation,
wherein the drug is in form of liposomes.

The virulent strain can be any virulent strain of MTB-C. One of the strains most used by researchers in this field is called H₃₇Rv which, for example, can be freely acquired in the National Collection of Type Cultures (NCTC), London, Great Britain (deposit number NC007416).

The virulent strain can be cultivated by inoculation in culture media well-known by the person skilled in the art, for example Middlebrook 7H10 or 7H11 agar, Sauton's medium or Proskauer-Beck medium.

The culture of the virulent strain is performed over a period equal to or greater than three weeks, preferably comprised between 3 and 4 weeks. The temperature of the culture is preferably maintained between 34° C and 38° C.

Once the culture ends, the cells are isolated using techniques such as those described, for example, in patent application ES2231037-A1.

The homogenisation of the live cells is carried out in the presence of a nonionic surfactant, and preferably in a buffered medium at neutral pH, for example at pH comprised between 6 and 8, such as that provided by PBS buffer (phosphate buffered saline).

The homogenisation can be carried out by means of ultrasound sonication, or by means of the use of small beads of approximately 1 mm in diameter, for example, silica or zirconia/silica beads, together with a mechanical homogeniser. A mechanical homogeniser that can be used, for example, is the BioSpec BeadBeater^{®} model.

The MTB-C cells are broken by means of this homogenisation process and small cell wall fragments are obtained.

The type of nonionic surfactant used in the homogenisation process is preferably selected from the group consisting of alkylphenol ethoxylates, sorbitan ester ethoxylates, and mixtures thereof.

More preferably, the nonionic surfactant is selected from the group of octylphenol ethoxylates. More preferably, octylphenol ethoxylates with an ethylene oxide content comprised between 7 and 8 moles are used, which surfactants can be found on the market under the name Triton X-114^{®}.

The nonionic surfactant content in the homogenisation step is preferably comprised between 1% and 10% by weight with respect to the total weight of the homogenate, more preferably between 3% and 6% by weight.

The homogenised mass containing the cell wall fragments is subjected to a conventional treatment to separate and reject the non-fragmented cells and the solubilized components. Centrifugation at different speeds and washing with buffer solution as described in patent application ES2231027-A1 can be used for example. Sediment containing the cell wall fragments is obtained after performing the mentioned purification processes. Said sediment is dispersed in PBS buffer and is subjected to a conventional treatment to ensure the complete inactivation of the MTB-C cells which may have remained viable after the fragmentation and purification process. The mentioned treatment can be a chemical process, for example by means of treatment with formaldehyde, or a physical process, for example by means of autoclaving or pasteurisation treatment.

The dispersion of cell wall fragments in PBS buffer obtained after the inactivation treatment can be lyophilised to facilitate the storage thereof. To that end, the dispersion can be distributed into vials and lyophilised at a temperature comprised between -15° C and -25° C and with a vacuum comprised between 0.1 and 0.5 mbar.

The vials obtained after the lyophilisation process contain the immunotherapeutic agent consisting essentially of the cell wall fragments of MTB-C, and they are generally stored at very low temperatures, for example at -70° C.

As previously indicated, the object of the invention is the use of the immunotherapeutic agent consisting essentially of cell wall fragments of a virulent strain of MTB-C for the preparation of a drug for the prophylactic treatment of tuberculosis, i.e., for the preparation of a prophylactic vaccine against tuberculosis.

The drug for the prophylactic treatment of tuberculosis consists essentially of the immunotherapeutic agent based on cell wall fragments The drug is in the form of liposomes.

The liposomes can be formed using conventional auxiliary lipids and techniques well-known by the person skilled in the art, such as those described in patent application ES2231037-A1.

The liposomes generally include phospholipids, with a neutral and/or negative net charge, and sterols.

The phospholipids used can be, for example: phosphatidylcholine, phosphatidylserine and phosphatidylinositol.

The main component of the liposomes is usually phosphatidylcholine, which can be synthesized or isolated from natural sources. A frequently used commercial product is soy-derived lecithin, which is a complex mixture of phospholipids including thereamong phosphatidylcholine.

The sterols which are used in the preparation of liposomes can be, among others, cholesterol and bile salts.

The liposomes are preferably formed using a mixture of soy-derived lecithin and sodium cholate.

The liposomes can optionally contain additives improving their stability, for example: vitamin E, which acts as a lipid antioxidant.

The liposomes obtained usually have a size distribution in which 99.9% are smaller than 1 micron.

The liposomes can be subjected to lyophilisation to thus obtain the immunotherapeutic agent in the form of lyophilised liposomes.

The drug can be administered in the form of a single dose or of several doses, by means of the repetition at certain time intervals. Preferably two doses are administered separated by a period comprised between 2 and 5 weeks, preferably between 3 and 4 weeks.

The drug can be administered in a mucosa, for example, ocular, intranasal, oral, gastric, intestinal, vaginal, or urinary tract mucosa, or parenterally, for example, subcutaneously, intradermally, intramuscularly, intravenously, or intraperitoneally. Parenteral administration is preferred.

The suitable dose depends on several parameters, including thereamong between the method of administration and the subject to be treated, but preferably the dose is comprised between 1 µg and 1000 µg, more preferably between 25 and 700, and even more preferably between 50 µg and 200 µg.

The drug consisting essentially of cell wall fragments of a virulent strain of MTB-C in form of liposomes can be administered in combination with other prophylactic vaccines against tuberculosis, such as those mentioned in S.H.E. Kaufmann, Nature Rev. Immunol. 2006, 6, 699-704, such as for example the BCG vaccine, subunit vaccines or recombinant BCG vaccines.

The combination of vaccines can be simultaneous or it can be done in two inoculations separated over time. The period between the inoculations can even be several years long.

In the case of inoculations separated over time, first a prophylactic vaccine against tuberculosis is preferably administered, and the drug consisting essentially of the cell wall fragments of a virulent strain of MTB-C, which acts as a re-stimulating (boost) agent of the initially inoculated vaccine, is subsequently administered.

It has surprisingly been found that the administration of the drug consisting essentially of the immunotherapeutic agent based on cell wall fragments of a virulent strain of MTB-C in the form of liposomes is able to induce a Th1 type interferon-γ generating response against *M. tuberculosis*-specific antigens. Said antigens include Ag85B and Ag85A, which are part of the complex Ag85, consisting of a family of low molecular weight proteins playing a decisive role in the biosynthesis of the cell wall and produced in considerable amounts when the bacterial cultivation is in the log phase.

It has been observed that the conventional BCG vaccine does not generate an immunoprotective response against antigens of complex Ag85, which could represent a lower protection capability.

It has also been found that the number of viable bacilli present in the lungs of mice vaccinated with said immunotherapeutic agent subsequently infected with the virulent H₃₇Rv strain is less than the number of viable bacilli present in the control mice group, and said number is comparable to that of the mice vaccinated with the conventional BCG vaccine.

The examples below are shown to provide the person skilled in the art with a detailed explanation of specific embodiments within the invention.

### Example 1.- Effectiveness of the immunotherapeutic agent as a prophylactic vaccine against

### the infection caused by M. tuberculosis

The immunotherapeutic agent used in this example was prepared according to the method described in Example 2 of patent application ES2231037-A1.

The effectiveness of the immunotherapeutic agent based on cell wall fragments of a virulent strain of MTB-C was assayed in C57BL/6 type female mice from 6 to 8 weeks of age and free of specific pathogens.

The mice were divided into three groups of 12 animals each and were subjected to the following vaccination protocol:
1) Without vaccination (control group),
2) Subcutaneously inoculated with two doses of 285 µg of the immunotherapeutic agent obtained in Example 2 of patent application ES2231037-A1 at weeks 3 and 6 of the experiment.
3) Subcutaneously inoculated with a dose of 2 x 10⁶ colony forming units of the BCG

Danish strain (Statens Serum Institute, Denmark) at week 0 of the experiment.

The virulent strain of *Mycobacterium tuberculosis* (H₃₇Rv Pasteur), which was cultivated in Proskauer-Beck medium until a mid-log phase, was used for the infection and it was stored in aliquots of 1 ml at a temperature of -70° C until its use.

The mice were aerosol-infected with said virulent strain at week nine of the experiment by means of placing them in a Middlebrook aerosol infection apparatus which provided an inoculum of approximately 10-50 viable bacilli in the lungs of the mice.

The number of viable bacilli in the lungs was determined 3 weeks after the aerosol infection of the animals (week 12 of the experiment) incubating serial dilutions of lung homogenate in Middlebrook 7H11 agar for 4 weeks at 37° C. The lung was homogenized in the presence of 1 ml of twice-distilled water.

The results of Table I express the logarithm of the colony forming units (CFU) per ml which have been identified in the lung:

**Table I**

| Group of mice | Vaccine | Log₁₀CFU/m 1 |
|---|---|---|
| 1 | None (Control Group) | 6.42±0.24 |
| 2 | Liposome-encapsulated immunotherapeutic agent | 5.72±0.30 |
| 3 | BCG | 5.71±0.58 |

The differences between the result of the group of mice which have not been vaccinated and the results of the groups vaccinated are statistically significant.

It can be observed that in the lung of the group of mice vaccinated with the liposome-encapsulated immunotherapeutic agent a smaller number of viable bacilli is detected than in the lung of the mice not vaccinated, and a substantially identical number to that of the mice vaccinated with the conventional BCG vaccine.

Therefore, the vaccination with the immunotherapeutic agent based on cell wall fragments of a virulent strain of MTB-C achieves protecting against infections caused by *M. tuberculosis.*

### Example 2.- Effectiveness of the immunotherapeutic agent as a generator of a Th1 response specific against infection caused by M. tuberculosis

The immunotherapeutic agent used in this example was prepared according to the method described in Example 2 of patent application ES2231037-A1.

The effectiveness of the immunotherapeutic agent based on cell wall fragments of a virulent strain of MTB-C was assayed in C57BL/6 type female mice from 6 to 8 weeks of age and free of specific pathogens in an *ex vivo* experiment.

The mice were divided into groups of 4 animals each and were subjected to the following inoculation schedule:
1) Subcutaneously inoculated with saline at weeks 3 and 6 of the experiment (control group),
2) Subcutaneously inoculated with two doses of 285 µg of the immunotherapeutic agent obtained in Example 2 of patent application ES2231037-A1 at weeks 3 and 6 of the experiment.
3) Subcutaneously inoculated with a dose of 2 x 10⁶ colony forming units of the BCG Danish strain (Statens Serum Institute, Denmark) at week 0 of the experiment, and with saline at weeks 3 and 6 of the experiment.
4) Subcutaneously inoculated with 2 x 10⁶ colony forming units of the virulent strain of *Mycobacterium tuberculosis* (H₃₇Rv Pasteur) at week 3 of the experiment, and with saline at week 6 of the experiment.

The mice were sacrificed at week 7, and their spleens were extracted and immersed in tubes containing 5 ml of L-glutamine-RPMI (Gibco). The tubes were kept in ice until the end of the experiment. The spleens were mechanically disintegrated and the suspension was filtered through a nylon mesh 70 µm in diameter. Then it was centrifuged for 10 minutes at 300 g. The pellets were reconstituted with 15 ml of a solution consisting of Tris and ammonium chloride in twice-distilled water to perform lysis on the cells. After 8 minutes they were washed with 20 ml of L-glutamine-RPMI and centrifuged for 10 minutes at 300 g.

The obtained pellets were resuspended with 5 ml of L-glutamine-RPMI, and the suspension was filtered through a nylon mesh 70 µm in diameter. The cells were counted with the Neubauer chamber.

The cells from the spleen of the mice were seeded on dishes at a ratio of 1 x 10⁵ cells/well and were cultivated with 200 µl of the complete culture medium (CCM) consisting of L-glutamine-RPMI supplemented with inactivated foetal calf serum, penicillin, streptomycin, sodium pyruvate, and 2-mercaptoethanol, or with 200 µl of complete culture medium (CCM) supplemented with *M. tuberculosis* antigens: 10 µg/ml of the antigen PPD (Statens Serum Institut, Denmark) and 5 µg/ml of ESAT-6, Ag85A and Ag85B (Lionex Diagnostics and Therapeutics GmbH, Federal Republic of Germany).

The cells were incubated at 37° C in an atmosphere with 5% CO₂. After 96 hours, the dishes were centrifuged for 10 minutes at 300 g, and the supernatant of each of them was collected.

The supernatants were frozen and after remaining in said state at least 24 hours, the interferon-γ content was analyzed applying the double sandwich ELISA technique and using a monoclonal antibody specific for murine interferon-γ (Diaclone).

Table II shows the average interferon-γ concentration expressed as log₁₀ pg/ml which was induced in each of the groups of mice against the *M. tuberculosis* antigens:

**TABLE II**

| Group | Inoculum | Antigens | | | | |
|---|---|---|---|---|---|---|
| | | Control | PPD | ESAT-6 | Ag85B | Ag85A |
| 1 | Control | 0 | 0 | 0 | 0 | 0 |
| 2 | Liposome-encapsulated immunotherapeutic agent | 0 | 2.57 | 0 | 2.69⁽¹⁾ | 2.53⁽¹⁾ |
| 3 | BCG | 0 | 2.01⁽³⁾ | 0 | 0 | 0 |
| 4 | H₃₇Rv | 0 | 3.06⁽³⁾ | 2.86⁽²⁾⁽³⁾ | 2.76⁽³⁾ | 2.50⁽³⁾ |

| | | | | | | |
|---|---|---|---|---|---|---|
| (1) Statistically significant differences between Group 2 and Group 3 (2) Statistically significant differences between Group 2 and Group 4 (3) Statistically significant differences between Group 3 and Group 4 | | | | | | |

The results of Table II show that the two inoculations performed with the liposome-encapsulated immunotherapeutic agent based on cell wall fragments of a virulent strain of *M*. *tuberculosis* are able to induce the production of interferon-γ against *M. tuberculosis*-specific antigens. This means that they induce a Th1 type protective response.

Particularly, the response induced by the group of mice inoculated with the liposome-encapsulated immunotherapeutic agent against the antigens PPD, Ag85B and Ag85A presents no significant differences with the response of the group of mice inoculated with the virulent H₃₇Rv strain of *M. tuberculosis.* Said response is better than the response of the group of mice inoculated with the BCG strain, which is the conventional vaccine, since it only induces a response against PPD and not against Ag85B and Ag85A.

Only the group of mice inoculated with the virulent strain was able to induce a response against the antigen ESAT-6.

Therefore, the immunotherapeutic agent based on cell wall fragments of a virulent strain of *M. tuberculosis* is able to induce a Th1 type protective response against *M. tuberculosis-*specific antigens, which is an indicator that it can be used effectively to prevent infections caused by *M. tuberculosis*.

## Claims

1. The use of an immunotherapeutic agent consisting essentially of cell wall fragments of a virulent strain of *Mycobacterium tuberculosis*-complex (MTB-C) for the preparation of a drug for the prophylactic treatment of tuberculosis, wherein said agent is obtainable by a method comprising the following steps:
- cultivate the virulent MTB-C strain over a period equal to or greater than three weeks and, subsequently,
- homogenate the cell culture in the presence of a nonionic surfactant.
- separating the non-fragmented cells and the solubilized components by means of centrifugation,
- subjecting the fraction of cell wall fragments to chemical or physical treatment to inactivate the eventual virulent strain cells that it eventually contains, and
- drying the obtained immunotherapeutic agent by means of lyophilisation, wherein the drug is in the form of liposomes.

2. The use according to claim 1, **characterised in that** the culture period is comprised between 3 and 4 weeks.

3. The use according to claims 1 or 2, **characterised in that** the nonionic surfactant is selected from the group consisting of alkylphenol ethoxylates, sorbitan ester ethoxylates, and mixtures thereof.

4. The use according to claim 3, **characterised in that** the nonionic surfactant is selected from the group of octylphenol ethoxylates.

5. The use according to claim 4, **characterised in that** the nonionic surfactant is selected from the octylphenol ethoxylates with an ethylene oxide content comprised between 7 and 8 moles.

6. The use according to any of claims 1 to 5, **characterised in that** the homogenisation is performed in a buffered medium at neutral pH.

7. The use according to any of claims 1 to 6, **characterised in that** the drug is to be administered in the form of a single dose or of several doses.

8. The use according to claim 7, **characterised in that** the drug is to be administered in two doses.

9. The use according to claim 8, **characterised in that** the doses are to be administered separated by a period comprised between 2 and 5 weeks.

10. The use according to any of claims 1 to 9, **characterised in that** the drug is to be administered in combination with other prophylactic vaccines against tuberculosis.

11. The use according to claim 10, **characterised in that** the vaccines are combined in two inoculations separated over time.

12. The use according to claim 11, **characterised in that** first a prophylactic vaccine against tuberculosis is to be administered, and the drug consisting essentially of the cell wall fragments of a virulent strain of MTB-C is subsequently administered.

## Patentansprüche

1. Verwendung eines immunotherapeutischen Mittels bestehend im wesentlichen aus Zellwandfragmenten eines virulenten Stamms von Mycobacterium tuberculosis-Komplex (MTB-C) zur Herstellung eines Arzneimittels für die prophylaktische Behandlung von Tuberkulose, wobei das Mittel durch ein Verfahren erhältlich ist, das folgende Schritte umfasst:
- Kultivieren des virulenten MTB-C-Stamms über einen Zeitraum von drei Wochen oder mehr, sowie anschließend
- Homogenisieren der Zellkultur in Gegenwart eines nicht-ionischen Tensids,
- Trennen der nicht-fragmentierten Zellen und der aufgeschlossenen Bestandteile durch Zentrifugation,
- Unterziehen der Fraktion von Zellwandfragmenten einer chemischen oder physikalischen Behandlung, um die eventuell vorhandenen Zellen des virulenten Stamms zu inaktivieren, die diese eventuell enthält, und
- Trocknen des erhaltenen immuntherapeutischen Mittels durch Lyophilisierung,
wobei das Arzneimittel in Form von Liposomen vorliegt.

2. Verwendung gemäß Anspruch 1, **dadurch gekennzeichnet, dass** die Kulturdauer im Zeitraum von 3 bis 4 Wochen eingeschlossen ist.

3. Verwendung gemäß Ansprüchen 1 oder 2, **dadurch gekennzeichnet, dass** das nicht-ionische Tensid ausgewählt wird aus der Gruppe bestehend aus Alkylphenolethoxylaten, Sorbitanesterethoxylaten und Mischungen davon.

4. Verwendung gemäß Anspruch 3, **dadurch gekennzeichnet, dass** das nicht-ionische Tensid ausgewählt wird aus der Gruppe der Octylphenolethoxylate.

5. Verwendung gemäß Anspruch 4, **dadurch gekennzeichnet, dass** das nicht-ionische Tensid ausgewählt wird aus den Octylphenolethoxylaten mit einem Ethylenoxidgehalt zwischen 7 und 8 mol.

6. Verwendung gemäß einem der Ansprüche 1 bis 5, **dadurch gekennzeichnet, dass** die Homogenisierung in einem gepufferten Medium bei neutralem pH durchgeführt wird.

7. Verwendung gemäß einem der Anasprüche 1 bis 6, **dadurch gekennzeichnet, dass** das Arzneimittel in Form einer einzigen Dosis oder von mehreren Dosen verabreicht werden soll.

8. Verwendung gemäß Anspruch 7, **dadurch gekennzeichnet, dass** das Arzneimittel in zwei Dosen verabreicht werden soll.

9. Verwendung gemäß Anspruch 8, **dadurch gekennzeichnet, dass** die Dosen getrennt durch einen Zeitraum zwischen 2 und 5 Wochen verabreicht werden sollen.

10. Verwendung gemäß einem der Ansprüche 1 bis 9, **dadurch gekennzeichnet, dass** das Arzneimittel in Kombination mit weiteren prophylaktischen Impfstoffen gegen Tuberkulose verabreicht werden soll.

11. Verwendung gemäß Anspruch 10, **dadurch gekennzeichnet, dass** die Impfstoffe in zwei zeitlich getrennten Impfungen kombiniert werden.

12. Verwendung gemäß Anspruch 11, **dadurch gekennzeichnet, dass** zuerst ein prophylaktischer Impfstoff gegen Tuberkulose verabreicht wird und anschließend das Arzneimittel, das im wesentlichen aus den Zellwandfragmenten eines virulenten Stammes von MTB-C besteht, verabreicht wird.

## Revendications

1. Utilisation d'un agent immunothérapeutique consistant essentiellement en des fragments de parois cellulaires d'une souche virulente de *Mycobacterium* complexe *tuberculosis* (MTB-C) pour la préparation d'un médicament destiné au traitement prophylactique de la tuberculose, dans lequel ledit agent peut être obtenu par un procédé comprenant les étapes suivantes :
- cultiver la souche virulente de MTB-C pendant une période supérieure ou égale à trois semaines, et, ensuite,
- homogénéiser la culture cellulaire en présence d'un tensioactif non ionique.
- séparer les cellules non fragmentées et les composants solubilisés au moyen d'une centrifugation,
- soumettre la fraction des fragments de parois cellulaires à un traitement chimique ou physique pour inactiver les cellules de la souche virulente éventuelles qu'elle contient éventuellement, et
- sécher l'agent immunothérapeutique obtenu au moyen d'une lyophilisation,
dans lequel le médicament est sous la forme de liposomes.

2. Utilisation selon la revendication 1, **caractérisée en ce que** la période de culture est comprise entre 3 et 4 semaines.

3. Utilisation selon les revendications 1 ou 2, **caractérisée en ce que** le tensioactif non ionique est choisi dans le groupe constitué par les éthoxylates d'alkylphénol, les éthoxylates d'ester de sorbitan, et les mélanges de ceux-ci.

4. Utilisation selon la revendication 3, **caractérisée en ce que** le tensioactif non ionique est choisi dans le groupe des éthoxylates d'octylphénol.

5. Utilisation selon la revendication 4, **caractérisée en ce que** le tensioactif non ionique est choisi parmi les éthoxylates d'octylphénol ayant une teneur en oxyde d'éthylène comprise entre 7 et 8 moles.

6. Utilisation selon l'une quelconque des revendications 1 à 5, **caractérisée en ce que** l'homogénéisation est effectuée dans un milieu tamponné à pH neutre.

7. Utilisation selon l'une quelconque des revendications 1 à 6, **caractérisée en ce que** le médicament doit être administré sous la forme d'une dose unique ou de plusieurs doses.

8. Utilisation selon la revendication 7, **caractérisée en ce que** le médicament doit être administré en deux doses.

9. Utilisation selon la revendication 8, **caractérisée en ce que** les doses doivent être administrées séparées par une période comprise entre 2 et 5 semaines.

10. Utilisation selon l'une quelconque des revendications 1 à 9, **caractérisée en ce que** le médicament doit être administré en combinaison avec d'autres vaccins prophylactiques contre la tuberculose.

11. Utilisation selon la revendication 10, **caractérisée en ce que** les vaccins sont combinés dans deux inoculations séparées dans le temps.

12. Utilisation selon la revendication 11, **caractérisée en ce que** d'abord un vaccin prophylactique contre la tuberculose doit être administré, et le médicament, constitué essentiellement des fragments de parois cellulaires d'une souche virulente de MTB-C est ensuite administré.
